# EUROPÄISCHE PATENTSCHRIFT

(11) **EP 0 847 755 B1**
(45) Veröffentlichungstag und Bekanntmachung des Hinweises auf die Patenterteilung: **19.09.2001**
(21) Anmeldenummer: 97121030.7
(22) Anmeldetag: 29.11.1997
(51) Int. Cl.: A61K 35/78, A61P 5/32

(54) **Verwendung eines Extraktes aus Cimicifuga racemosa**
Use of an extract of cimicifuga racemosa
Utilisation d'un extrait de cimicifuga racemosa

(30) Priorität: 14.12.1996 DE 19652183
(43) Veröffentlichungstag der Anmeldung: 17.06.1998
(73) Patentinhaber: SCHAPER & BRÜMMER GMBH & CO. KG, D-38259 Salzgitter (DE)
(72) Erfinder: Nesselhut, Thomas Dr., 37075 Göttingen (DE); Bodinet, Cornelia, 38259 Salzgitter (DE); Schneider, Peter Dr., 38259 Salzgitter (DE); Freundenstein, Johannes Dr., 38640 Goslar (DE)
(74) Vertreter: Lins, Edgar, Dipl.-Phys. Dr.jur.

(56) Entgegenhaltungen:
- PATENT ABSTRACTS OF JAPAN vol. 015, no. 001 (C-0793), 7.Januar 1991 & JP 02 255622 A (TSUMURA & CO), 16.Oktober 1990,
- T. NESSELHUT ET AL.: "UNTERSUCHUNGEN ZUR PROLIFERATIVEN POTENZ VON PHYTOPHARMAKA MIT ÖSTROGENÄHNLICHER WIRKUNG BEI MAMMAKARZINOMZELLEN." ARCHIVES OF GYNECOLOGY AND OBSTETRICS, Bd. 254, Nr. 1-4, 1993, Seiten 817-818, XP002060552

## Beschreibung

Die Erfindung betrifft die Verwendung eines Extraktes aus Cimicifuga racemosa zur Herstellung eines Arzneimittels zur Behandlung östrogenabhängiger Tumore.

Extrakte aus dem Wurzelstock der Traubensilberkerze (Cimicifugae racemosae rhizoma) weisen östrogenähnliche Effekte auf. In den Extrakten sind Komponenten gefunden worden, die spezifisch an Östrogenrezeptoren binden und bei ovarektomierten Ratten die Gonadotropinspiegel senken können. Die Verabreichung dieser Extrakte zur Behandlung klimakterischer Beschwerden und Dysmenarrhö hat sich daher bewährt.

Für Mammakarzinom-Risikopatienten verbietet sich die Anwendung von östrogenhaltigen Arzneimitteln zur Regulierung von klimakterischen Beschwerden, da die Ausbreitung von östrogenabhängigen Tumoren naturgemäß durch Östrogengaben verstärkt wird. Da der Mechanismus der Wirkungsweise für die östrogen-analogen Substanzen noch unklar ist, ist vorsorglich für die Verabreichung dieser Substanzen ein Risiko für östrogenabhängige Tumore als Kontraindikation angesehen worden.

Es ist zwar bereits berichtet worden (Nesselhut et al. in TW Gynokologie (1993) Seiten 249 bis 250), daß die Phytopharmaka Rhaponticin und Cimicifuga-Extrakt in niedrigeren Konzentrationen die Proliferation von Karzinomzellen in vitro verstärken, in höheren Konzentrationen hingegen möglicherweise hemmen.

Eine Verifizierung dieser Ergebnisse ist nicht publiziert worden, so daß das Verbot der Verabreichung von Phytopharmaka mit östrogen-analoger Wirkung für Risikopatienten bezüglich östrogenabhängiger Tumore weiterhin besteht.

Es ist bekannt, östrogenabhängige Tumore mit einem antiöstrogenen Wirkstoff zu therapieren. Der gängigste Wirkstoff dieser Art ist derzeit Tamoxifen (Z)-2-[4-(1,2-Diphenyl-1-Butenyl) Phenoxy]-N,N-Dimethylethylamin).

Für die Patienten, die mit einem derartigen Antiöstrogen behandelt wurden, kam aus den oben erwähnten Gründen eine Regulierung der klimakterischen Beschwerden durch Östrogene oder östrogenanaloge Substanzen nicht in Betracht.

Eine Hemmung der Proliferation von Mammatumorzellen ist abhängig von der Konzentration des Tamoxifens. Der Erhöhung der Konzentration in einen Bereich hinein, wo die Proliferation der Tumorzellen sicher verhindert wird, ist jedoch nicht möglich, da Tamoxifen in diesen Konzentrationen toxisch wird.

Die vorliegende Erfindung geht daher von der Problemstellung aus, eine Tumortherapie auch mit niedrigeren Antiöstrogen-Konzentrationen zu ermöglichen.

Erfindungsgemäß wird hierzu in Kombination mit dem Antiöstrogenen Wirkstoff ein Extrakt aus Cimicifuga racemosa verwendet.

Überraschenderweise hat sich herausgestellt, daß der Extrakt aus Cimicifuga racemosa die Proliferation von östrogenabhängigen Tumorzellen nicht nur nicht verstärkt sondern in Kombination mit einem antiöstrogenen Wirkstoff dessen poliferationshemmende Wirkung deutlich verstärkt, so daß auch eine vollständige Proliferationshemmung erreichbar ist, ohne in den Bereich der Toxizitätsgrenze für den antiöstrogenen Wirkstoff geraten zu müssen.

Die Potenzierung der Wirkung eines antiöstrogenen Wirkstoffes ist anhand des Standard-Wirkstoffes Tamoxifen genauer untersucht worden. Andere Experimente geben Hinweise darauf, daß auch die antiöstrogene Wirkung von Genistein durch einen Extrakt aus Cimicifuga racemosa verstärkt wird. Bevorzugte Verdünnungen des Extraktes liegen im Bereich zwischen 10⁻³ und 10⁻⁵, da Verdünnungen bis 10⁻² in vitro toxische Effekte hervorbringen. Bevorzugte Dosierungen liegen zwischen etwa 5 mg und 500 mg Droge pro Tag.

Die erfindungsgemäße Wirkung des Cimicifuga-Extraktes auf die Proliferation von östrogenabhängigen Karzinomzellen, insbesondere Mammakarzinomzellen, ist in vitro mit einem Testsystem aus MCF-7 Zellen erfolgt.

Die MCF-7 Zellinie ist ein etabliertes in vitro Modell für östrogenabhängige Tumore, die sowohl Östrogenrezeptoren als auch Aromataseaktivität besitzen. Die menschliche Brustkrebslinie wurde abgeleitet von einer Pleuraeffusion bei einem metastasierenden Brusttumor und besitzt signifikante Mengen an 17-β Rezeptoren (Schwarte, A. (1994) Wirkspektrum ausgewählter Flavonoide auf die humane Brustkrebslinie MCF-7: Eine in vitro Studie. Witten-Herdecke, Universität, Bereich Medizin, Diss. 1994).

Der Einfluß von Cimicifuga-Extrakt auf die Proliferation der MCF-7 Zellen wurde anhand des Einbaus von radioaktiv markiertem Thymidin bestimmt.

### Material und Methoden

### Testsubstanzen

17β-Estradiol (Sigma) und Tamoxifencitrat (Sigma) wurden 1-M in DMSO (Dimethylsulfoxid) gelöst und in einer 1:10 Verdünnungsreihe in Zellkulturmedium entsprechend weiter verdünnt. Cimicifuga-Extrakt wurde in einer 1:10 Verdünnungsreihe direkt mit Zellkulturmedium verdünnt.

### Herstellung des Cimicifuga racemosa-Extraktes

Nach Prüfung auf Identität, Reinheit und Gehalt wurden 30 kg der zerkleinerten Arzneidroge Cimicifuga racemosa rhizoma für 10 Tage mit 50 1 Isopropylalkohol 40 % (V/V) mazeriert. Der Extrakt wurde abgelassen und die Droge abgepreßt. Die vereinigten Fraktionen wurden mit Isopropylalkohol 40 % (V/V) auf ein Endvolumen von 35 l aufgefüllt.

### Proliferationsassay MCF-7

MCF-7 Zellen wurden von der ATCC (HTB 22) bezogen und in Eagle's MEM (Eagle's Minimal-Essential-Medium) mit nicht-essentiellen Aminosäuren, 1mM Natriumpyruvat, 10 µg/ml Insulin und 10 % FKS (Foetales Kälberserum) kultiviert.

Vor Einsatz in den Test wurden die MCF-7 Zellen mindestens eine Passage in Eagle's MEM ohne Phenolrot mit nicht-essentiellen Aminosäuren, 1 mM Natriumpyruvat, 10 µg/ml Insulin und 5 % FKS (Foetales Kälberserum) gehalten. Um östrogenfreie Wachstumsbedingungen im Test zu erhalten, wurde für den Testansatz das Insulin aus dem Zellkulturmedium entfernt und das FKS durch 5 % "Characoal stripped" FKS (CSF) ersetzt.

200 µl einer auf 5x10⁴ c/ml eingestellten Zellsuspension wurden pro Vertiefung in 96er Mikrotiterplatten (Nunc) pipettiert und 24 h bei 37 °C und 5 % CO₂ im Brutschrank inkubiert. Danach wurden die Überstände vorsichtig abgenommen und 150 µl frisches Zellkulturmedium pro Vertiefung pipettiert.

Die Testsubstanzen wurden gelöst, in Zellkulturmedium verdünnt und in 4 Parallelen a 50 µl/Vertiefung pipettiert. Als Kontrollen wurden Zellkulturmedium sowie die entsprechenden Lösungsmittelverdünnungen jeweils mitinkubiert.

Nach 2-3 Tagen Inkubation bei 37 °C und 5 % CO₂ wurden die Zellen mit 25 µl/Napf (6-³H)-Thymidin (Amersham, spez. Aktivität 2 Ci/mMol) für 8 h gepulst. Danach wurden sie nach Standardmethoden (Cell Harvester Inotech) auf Glasfaserfilter geerntet und in einem Flüssig-Szintillations-Counter (Wallac) gezählt. Die Ergebnisse wurden als cpm = "counts per minute" ausgedruckt.

### Herstellung des "Charcoal-Stripped" FKS (CSF)

1 Tablette "Dextran coated charcoal tablets"(Steranti Separe ₓ ®) wurde in 10 ml FKS aufgelöst. Danach wurde das Serum 2x45 min im Wasserbad bei 56 °C inaktiviert und im Anschluß bei 3000 rpm 10 min zentrifugiert. Der Überstand wurde abgenommen und über einen Filter mit Porengröße 0,2 µm filtriert.

### Toxizitätsassay

Zur Bestimmung der Toxizität der einzelnen Prüfsubstanzen wurde ein Fluoreszenzassay mit Hela Suspensionszellen durchgeführt.

Hela-Zellen wurden auf eine Zelldichte von 2,5x10⁵ c/ml Medium (Eagle's MEM + 5 % FKS) eingestellt und 100 µl der Suspension in 96er Mikrotiterplatten (Nunc) pipettiert. Nach 24 h Inkubation bei 37 °C und 5 % CO₂ wurde eine 1:2 Verdünnungsreihe der Testsubstanzen in Medium angelegt und von jeder Verdünnung jeweils 100 µl pro Vertiefung in 4 Parallelen pipettiert. Die Ansätze wurden 48 h bei 37 °C und 5 % CO₂ inkubiert. Danach wurden die Mikrotiterplatten bei 800 rpm zentrifugiert und die Überstände vorsichtig abgenommen. 200 µl einer Lösung aus 0,1 mg/ml 4-Methylumbelliferylheptanoat in PBS wurden pro Vertiefung pipettiert. Nach 60 min wurden die Fluoreszenzeinheiten pro Vertiefung in einem Mikrotiterplattenfluorimeter (Fluoroskan II) bestimmt.

### Ergebnisse

Zu Beginn der Untersuchungen wurde zunächst das Testsystem auf seine Sensitivität überprüft. Als Positivkontrolle wurde 17-β Östradiol in Dosierungen von 10⁻⁷, 10⁻⁸ bzw. 10⁻⁹-Molar getestet.

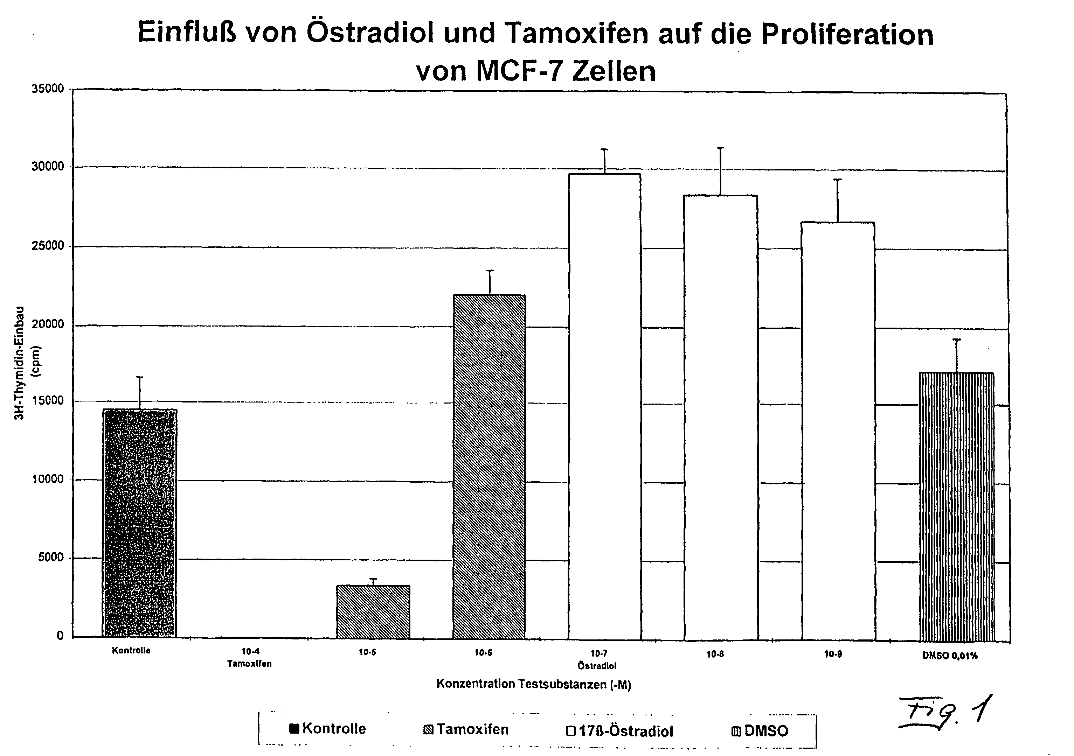

In allen drei getesteten Dosierungen induzierte 17-β Östradiol eine Steigerung der Proliferation von MCF-7 Zellen um 80-100 % im Vergleich zur unbehandelten Kontrolle, wie aus Figur 1 ersichtlich ist.

Parallel wurde in jedem Testansatz eine Lösungsmittelkontrolle durchgeführt. DMSO bewirkte in der maximal im Test eingesetzten Konzentration keine signifikante Proliferationssteigerung oder -hemmung im Vergleich zur unbehandelten Kontrolle.

In einem weiteren Ansatz wurde der Einfluß des nicht-steroidalen Antiöstrogens Tamoxifen auf das Testsystem geprüft.

Tamoxifen bewirkte in den Dosierungen 10⁻⁴ und 10⁻⁵-Molar eine 100%ige bzw. 77%ige Hemmung der Proliferation, in Dosierungen von 10⁻⁶ dagegen eine Steigerung der Einbauraten um 52 % im Vergleich zur unbehandelten Kontrolle.

Die antiproliferative Wirkung von Tamoxifen zeigte sich auch, wenn Tamoxifen zusammen mit einer konstanten Dosis an Östradiol appliziert wurde. Die östrogeninduzierte Proliferationssteigerung konnte dosisabhängig durch Tamoxifen inhibiert werden.

Die Ergebnisse dieser Vorversuche machten deutlich, daß das MCF-7 Testsystem geeignet ist, sowohl östrogene als auch antiöstrogene Wirkungen von Testsubstanzen nachzuweisen.

Um dies zu kontrollieren, wurden in den folgenden Versuchsserien neben Mediumkontrollen und Lösungsmittelkontrollen (Negativkontrollen) jeweils eine Positivkontrolle für östrogene Wirkung, bestehend aus 17β-Östradiol in einer Dosierung von 10⁻⁷ oder 10⁻⁸-Molar und eine Positivkontrolle für antiöstrogene Wirkung, bestehend aus Tamoxifen in einer Dosierung von 10⁻⁵-Molar, mitgeführt.

Bevor der Cimicifuga-Extrakt in das MCF-7 System eingesetzt wurde, wurde dessen Toxizität zunächst in einem Toxizitätsassay mit Hela-Zellen überprüft.

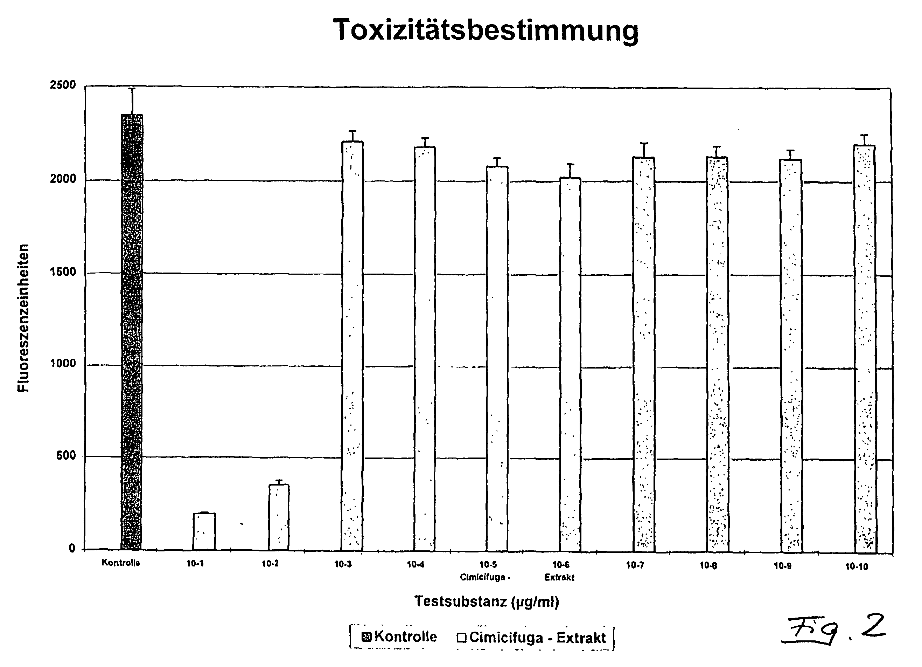

Bis zu einer Verdünnung von 10⁻² zeigte der Extrakt auf dieser Zellinie toxische Effekte. Ab einer Verdünnung von 10⁻³ waren keine Unterschiede zwischen Mediumkontrolle und Testansatz feststellbar (Fig. 2). Um unspezifische cytotoxische Effekte ausschließen zu können, wurde diese Verdünnung daher als Maximaldosis für die Testserien auf MCF-7 Zellen eingesetzt.

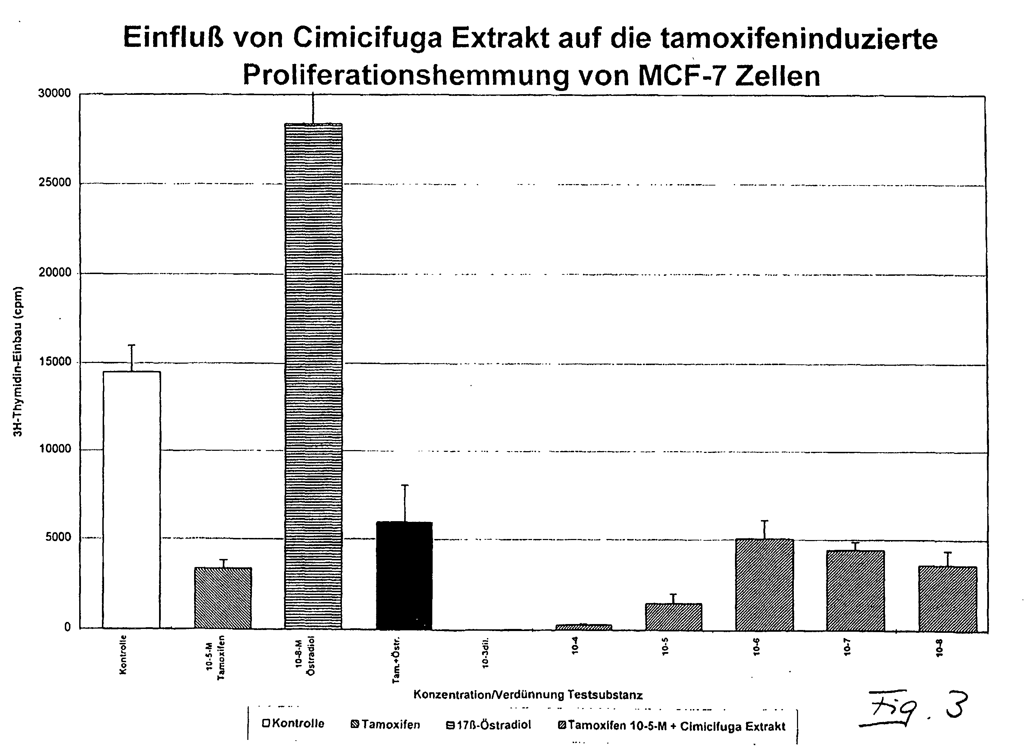

Figur 3 verdeutlicht im Vergleich die Proliferation für das Kontrollsystem, für eine 10⁻⁵ molare Tamoxifen-Lösung, eine 10⁻⁸ molare Östradiollösung sowie für eine Kombination von Tamoxifen und Östradiol, um auch die durch Tamoxifen verursachte Hemmung von östrogeninduzierten Proliferationen zu verifizieren.

Figur 3 zeigt ferner die Proliferationshemmung für Kombinationen von 10⁻⁵ molarem Tamoxifen mit Extrakt von Cimicifuga racemosa in verschiedenen 1:10-Verdünnungen zwischen 10⁻³ und 10⁻⁸. Es zeigt sich, daß in den Verdünnungen 10⁻³ bis 10⁻⁵ die proliferationshemmende Wirkung von Tamoxifen deutlich verstärkt wird, wobei in der Verdünnung 10⁻³ die Proliferation vollständig und in der Verdünnung 10⁻⁴ fast vollständig unterdrückt wird. In Verdünnungen von 10⁻⁶ und höher wird die proliferationshemmende Wirkung von Tamoxifen nicht verstärkt.

Durch die Kombination von Tamoxifen mit dem Cimicifuga-Extrakt in einer Verdünnung von 10⁻² oder 10⁻⁶ läßt sich daher eine nahezu vollständige Proliferationshemmung der Tumorzellen erreichen, ohne hierfür höhere Tamoxifen-Konzentrationen, beispielsweise 10⁻⁴-Molar einsetzen zu müssen, die bereits toxische Effekte zeigen.

## Patentansprüche

1. Verwendung eines Extraktes aus Cimicifuga racemosa zur Herstellung eines Arzneimittels zur Behandlung östrogenabhängiger Tumore in Kombination mit einem einen antiöstrogenen Wirkstoff enthaltenden Arzneimittel.

2. Verwendung eines Extraktes aus Cimicifuga racemosa in Kombination mit einem antiöstrogenen Wirkstoff zur Herstellung eines Arzneimittels zur Behandlung östrogenabhängiger Tumore.

3. Verwendung eines Extraktes nach Anspruch 1 oder 2 in Kombination mit Tamoxifen.

4. Verwendung eines Extraktes nach Anspruch 1 oder 2 in Kombination mit Genistein.

5. Verwendung eines Extraktes aus Cimicifuga racemosa nach einem der Ansprüche 1 bis 4 in einer Dosierung zwischen etwa 5 mg und etwa 500 mg Droge pro Tag.

## Claims

1. Use of an extract of *Cimicifuga racemosa* for the production of a pharmaceutical preparation for the treatment of oestrogen-dependent tumours in combination with a pharmaceutical preparation containing an anti-oestrogenic active substance.

2. Use of an extract of *Cimicifuga racemosa* in combination with an anti-oestrogenic active substance for the production of a pharmaceutical preparation for the treatment of oestrogen-dependent tumours.

3. Use of an extract according to claim 1 or 2 in combination with tamoxifen.

4. Use of an extract according to claim 1 or 2 in combination with genistein.

5. Use of an extract of *Cimicifuga racemosa* according to one of claims 1 to 4 at a dosage of between approx. 5 mg and approx. 500 mg of drug per day.

## Revendications

1. Utilisation d'un extrait de Cimicifuga racemosa pour la fabrication d'un médicament pour le traitement d'une tumeur oestrogènes dépendante en combinaison avec un médicament renfermant un agent actif anti-oestrogènes.

2. Utilisation d'un extrait de Cimicifuga racemosa en combinaison avec agent actif anti-oestrogène pour la fabrication d'un médicament pour le traitement d'une tumeur oestrogènes dépendante.

3. Utilisation d'un extrait selon la revendication 1 ou 2 en combinaison avec du Tamoxifène.

4. Utilisation d'un extrait selon la revendication 1 ou 2 en combinaison avec du Genistein.

5. Utilisation d'un extrait de Cimicifuga racemosa selon l'une des revendications 1 à 4 en un dosage d'environ 5mg et d'environ 500 mg de drogue par jour.
